# EUROPEAN PATENT SPECIFICATION

(11) **EP 1 824 485 B1**
(45) Date of publication and mention of the grant of the patent: **19.09.2012**
(21) Application number: 05823037.6
(22) Date of filing: 04.11.2005
(51) Int. Cl.: A61K 31/475, A61K 9/20, A61K 47/18

(54) **PHARMACEUTICAL FORMULATION FOR INCREASING SOLUBILITY OF 10-HYDROXYCAMPTOTHECIN COMPOUNDS IN NON-AQUEOUS POLAR SOLVENTS**
PHARMAZEUTISCHE FORMULIERUNG ZUR ERHÖHUNG DER LÖSLICHKEIT VON 10-HYDROXYCAMPTOTHECIN-VERBINDUNGEN IN NICHTWÄSSRIGEN POLAREN LÖSUNGSMITTELN
FORMULATION PHARMACEUTIQUE PERMETTANT D'AUGMENTER LA SOLUBILITE DE COMPOSES DE 10-HYDROXYCAMPTOTHECINE DANS DES SOLVANTS POLAIRES NON AQUEUX

(30) Priority: 05.11.2004 KR 20040089876
(43) Date of publication of application: 29.08.2007
(73) Proprietor: Samyang Biopharmaceuticals Corporation, Seoul 110-725 (KR)
(72) Inventor: SEO, Min-hyo, Daejeon 302-781 (KR); KANG, Hye-won, Daejeon 305-348 (KR)
(74) Representative: Wichmann, Hendrik
(86) International application number: PCT/KR2005/003706
(87) International publication number: WO 2006/049447

(56) References cited:
- WO-A-00/59475
- WO-A-2004/087115
- US-A- 5 447 936
- US-A- 5 674 874
- US-A- 5 859 023
- US-A- 5 859 023
- US-A- 5 900 419
- V. HERBEN ET AL.: "Sensitive determination of the carboxylate and lactone forms of the novel antitumor drug irinotecan and its active metabolite in plasma by HPLC" JOURNAL OF LIQUID CHROMATOGRAPHY AND RELATED TECHNOLOGIES, vol. 21, no. 10, 1998, pages 1541-1558, XP008084504
- R. ADELMAN: "Studies on the base strength of N,N-disubstituted amides", JOURNAL OF ORGANIC CHEMISTRY, vol. 29, no. 7, 1964, pages 1837-1844,

## Description

### TECHNICAL FIELD

The present invention relates to a pharmaceutical formulation comprising of a 10-hydroxycamptothecin compound and an amine compound whose pKₐ value is 7.4 or more for increasing the solubility of the 10-hydroxycamptothecin compound in non-aqueous polar solvent, as defined in the claims.

### BACKGROUND ART

Camptothecin; (S)-4-Ethyl-4-hydroxy-1H-pyrano[3',4':6,7]indolizino[1,2-b]quinol ine-3,14(4H,12H)-dione) is prototype DNA topoisomerase 1 inhibitor, and is known as strong anti-cancer medicine. Camptothecin has such standard structure that hydroxyl and ethyl are substituted at a position of C20 in the central structure containing five connected rings A to E, as shown in the following formula 1:

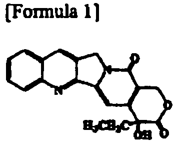

Camptothecin compound has very low solubility in water as well as in organic solvents, such as ethanol, dichloromethane, acetone, acetonitrile, ethylacetate, etc. which are generally used for formulation. Also, camptothecin is activated in acidic conditions since the E ring thereof exists in the form of a lactone, whereas it is inactive in alkaline aqueous solution since the E ring exists in the inactive form of a carboxy anion. The solubility of the inactive form of the carboxy anion in water is 4 mg/ml or more, but that of the active form of lactone form is not more than 10 µg/ml.

Particularly, 10-hydroxycamptothecin (compound of formula la) having a phenolic hydroxyl group at 10 position of A ring is ionized in basic solution whose pH is 8 to 12, and thus the solubility of the camptothecin compound in polar solvent can be increased. However, if the above basic solution is an aqueous solution, a hydroxyl ion attacks the lactone ring of the camptothecin compound, thereby forming camptothecin compound of formula 2 in the form of carboxy anion, and being converted to the inactive form (see Reaction equation 1):

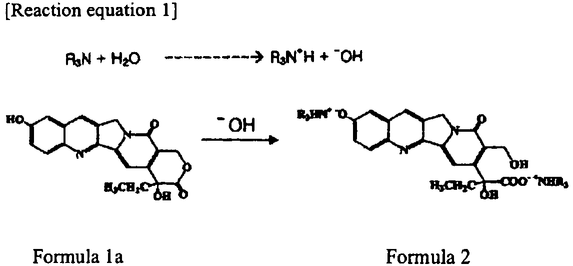

As shown above, camptothecin compound has highly potent anti-cancer activity, but its use for anti-cancer therapy is limited since the active form of camptothecin compound has very low solubility in water. Thus, in order to use camptothecin compound as a medicine for treating diseases, it is needed to increase the solubility of the camptothecin compound in solvent with maintaining the lactone structure, i.e., active form.

For example, 7-ethyl-10-hydroxycamptothecin (compound of formula 1b) is known as SN-38, and is an active metabolite of Irinotecan (CPT-11) which is an anti-cancer agent on the market.

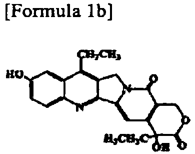

SN-38 is known to kill cells by combining with topoisomerase I , an enzyme participating in the process of cell division, and by inhibiting synthesis of DNA during cell division. However, the solubility of SN-38 in water is not more than 10 ug/ml, and as such it is difficult to develop SN-38 as a clinic product. Thus, CPT-11 which increases the solubility of SN-38 in water by making it as a prodrug has been developed and commercialized. After administration into the human body, CPT-11 is metabolized by carboxyesterase in the liver or cancer cell, and converted into SN-38 having physiological activity to show anti-cancer effects. However, the conversion ratio of CPT-11 into SN-38 having such activity in the human body is only about 2 to 8%.

On the other hand, it has been found that the activity of SN-38 for inhibiting topoisomerase I is higher than that of CPT-11 by about 1,000 times, and the *in vitro* cytotoxicity of SN-38 is higher than that of CPT-11 by 2,000 times. Also, it is known that the effect of SN-38 for inhibiting acetylcholine which causes diarrhea is much lower than that of CPT-11, and thus the possibility to cause diarrhea that is one of the main side effects of CPT-11 is very low.

SN-38 exists in the active form of a lactone in acidic conditions, and exists in the inactive form of a carboxy anion in alkaline condition, depending on pH in aqueous solution. It is known that the solubility in water of SN-38 in the form of the carboxy anion is 4 or more mg/ml, but that of SN-38 in the active form of lactone is not more than 10 µg/ml. Thus, if camptothecin compounds can be solubilized at a more than clinically significant concentration while maintaining the active form of a lactone, they can be developed as a very superior anti-cancer agent. Therefore, there have been extensive studies to solubilize camptothecin compounds. For example, U.S. Patent Nos. 5,447,936, 5,859,023, 5,674,874, 5,958,937, and 5,900,419 disclosed compositions to solubilize camptothecin compounds comprising SN-38 in polar organic solvents such as dimethylaceteamide, N-methyl-2-pyrrolidone, dimethylisosorbide, etc. However, the injection of such polar organic solvent into blood vessels is extremely limited due to some drawbacks that the amount of solvent usable for the human body is limited and the drug is extracted at the time of mixing with water. Also, U.S. published patent No. 2003-215492 disclosed a liposome-formulated composition by complexing SN-38 with lipid. The method used therein is to form SN-38 in the form of a lactone under the pH of acidic conditions, after forming SN-38 in the form of a carboxy anion in an aqueous solution at pH 8 ~ 10 and formulating it into liposome. Also, Zhang, et al. [International Journal of Pharmaceutics, 270 (2004), pp. 93-107] disclosed a LE-SN-38 liposome formulation containing SN-38, and Williams, et al. [Journal of Controlled Release, 91 (2003), pp. 167-172] disclosed a nanoparticle formulation containing SN-38.

WO 2004/087115 discloses delivery systems using liposomal vehicles that are stably associated with at least fluoropyrimidine and one water-soluble camptothecin.

Herben et al. (J. Liq Chrom. & Rel. Technol., 21(10): 1541-1558 (1998)) describe a sensitive high-performance liquid chromatography (HPLC) method for the determination of the lactone and carboxylate forms of the antitumor drug irinotecan (CPT-11) and its active metabolite SN-38 in plasma after intravenous infusion of CPT-11.

WO 00/59475 describes compositions and methods for improved delivery of ionisable hydrophobic therapeutic agents, which are in particular suitable for use in oral dosage forms.

However, the need remains to develop a method of solubilizing 10-hydroxycamptothecin at a more than clinically significant concentration while maintaining the active form.

### DISCLOSURE OF THE INVENTION

### TECHNICAL SUBJECT

The present inventors have conducted continuous studies to develop a method for solubilizing a 10-hydroxycamptothecin compound at a more clinically significant concentration while maintaining the active form. As a result, they confirmed that if an amine compound having a pKₐ value of 7.4 or more is added to a 10-hydroxycamptothecin, the base's nucleophilic attack to the lactone group of the camptothecin compound docs not happen, and thus the lactone group is conserved, maintaining the drug's activity, and also the hydroxyl group at the 10-position is ionized to a phenolic anion, and so the solubility in polar organic solvent can be increased. Therefore, the present inventors completed the present invention.

The object of the present invention is to provide a formulation for increasing the solubility of a 10-hydroxycamptothecin compound comprising the 10-hydroxycamptothecin compound and an amine compound whose pKₐ value is 7.4 and more, for non-aqueous polar solvent, as defined in the claims.

### BEST MODE FOR CARRYING OUT THE INVENTION

First, the present invention relates to an injectable formulation for increasing the solubility of a 10-hydroxycamptothecin compound in non-aqueous polar solvent comprising the 10-hydroxycamptothecin compound, an amine compound whose pKₐ value is 7.4 or more, and a non-aqueous polar solvent, as defined in the claims.

In the present invention, a 10-hydroxycamptothecin compound means all camptothecin compounds having a phenolic hydroxy group at 10 position of A ring of the camptothecin as shown in the following formula 1a:

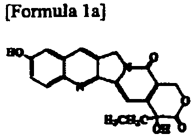

A 10-hydroxycamptothecin compound according to the present invention can be additionally substituted with lower alkyl, alkoxy, acyloxy, hydroxy, acyl, halo, amido, cyano group, etc. at 9 or 11 position of A ring, or 7 position of B ring. The 10-hydroxycamptothecin compounds of the present invention comprises, but is not limited to, 10-hydroxycamptothecin and 7-ethyl-10-hydroxy camptothecin(SN-38). In the present invention, 10-hydroxycamptothecin is preferably SN-38.

According to the present invention, if a 10-hydroxycamptothecin compound, for example SN-38, is added to non-aqueous polar solvent together with an amine compound that has a particularly low nucleophilic and high basic property, that is, the pKₐ value of the compound is 7.4 or more, among basic compounds, the base's nucleophilic attack to the lactone group of the camptothecin compound does not happen, as shown in the following Reaction equation 1, and so the lactone group is conserved. Then, while the activity of a drug is maintained, the hydroxyl group at the 10-position is ionized to a phenolic anion, to increase the solubility in non-aqueous polar solvent (see Reaction equation 2).

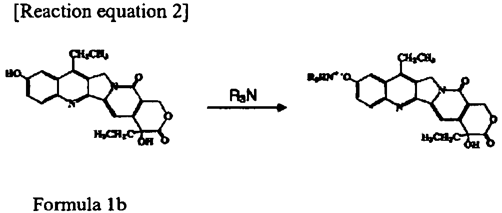

An amine compound of the present invention can anionize the hydroxy group at the 10 position, and is a primary, secondary, or tertiary amine compound whose pKₐ value is 7.4 or more, preferably 9 or more. Such amine compounds include hydroxyalkylamines such as mono-, di-, or triethanolamine, mono-, di-, or triisopropanolamine, tromethamine(tris(hydroxymethyl) aminoethane) ; tertiary alkylamines such as triethylamine, tripropylamine, tributylamine ; secondary alkylamines such as diethylamine, dipropylamine, dibutylamine ; or mixtures thereof, preferably, hydroxyalkylamines such as diethanolamine, triethanolamine, tromethamine.

Amine compounds can be used in an amount of more than 1 equivalent to I equivalent of camptothecin compound, preferably 1 to 100 equivalents, more preferably 1 to 50 equivalents, most preferably 5 to 20 equivalents.

The non-aqueous polar solvent of the present invention includes organic solvents approved for use in the human body: ethanol, propyleneglycol, liquid polyethyleneglycol having a molecular weight of 200 to 1,000 dalton, glycerine, N,N-dimethylacetamide, N-methylpyrrolidone, dimethylisosorbide, or mixtures thereof. The above non-aqueous polar solvent is used in an amount enough to make the concentration of 10-hydroxycamptothecin, preferably 0.1 to 5 mg/ml, and more preferably 0.5 to 2 mg/ml.

The formulation composition of the present invention may contain surfactant in order to prevent the precipitation of drug when the composition is diluted or reconstituted with aqueous solution such as water for injection, physiological saline, or 5% dextrose solution, and to delay the conversion of the lactone group to the carboxy anion by postponing the exposure of the lactone group of camptothecin compound to aqueous solution.

As a surfactant of the present invention, any surfactant can be used regardless of property and appearance thereof, when the present formulation composition is solubilized in organic solvent. The examples of such surfactant include block copolymer surfactants such as polyoxyethylenesorbitan fatty acid ester like tween 20, tween 40, tween 80, etc.; polyoxyethylene castor oil derivatives such as cremophore EL, cremophore RH40, cremophore RH60, etc.; polyoxyethylene alkyl ethers such as polyoxyl 20 cetosteryl ether, polyoxyl 20 cetyl ether, polyoxyl 20 oleyl ether, polyoxyl 2 cetyl ether, polyoxyl 2 oleyl ether, polyoxyl 2 stearyl ether, polyoxyl 4 lauryl ether, polyoxyl 100 stearyl ether, etc.; d-α-tocopheryl polyethyleneglycol 1000 succinate (vitamine E TPGS), solutol HS 15 (polyethyleneglycol 660 12-hydroxystearate); poloxamers such as poloxamer 124, poloxamer 188, poloxamer 407, etc., preferably polyoxyethylenesorbitan fatty acid ester, polyoxyethylene castor oil derivative, d-α-tocopheryl polyethyleneglycol 1000 succinate, solutol HS 15, etc.

On the other hand, when the present formulation composition is prepared in lyophilized form, the present invention may use polymer surfactants which are solid at room temperature, block copolymers of poloxamer such as poloxamer 124, poloxamer 188, poloxamer 407, etc., or amphiphilic block copolymers prepared by connecting hydrophilic polymer block (A) and hydrophobic polymer block (B) in form of A-B, A-B-A, B-A-B, etc. In amphihilic block copolymers, hydrophilic polymer block (A) is an aqueous soluble polymer, for example, polyalkyleneglycol or derivatives thereof, polyvinylalcohol, polyvinylpyrrolidone or polyacrylamide, etc., preferably, one of polyethyleneglycol, polyvinylpyrrolidone, and polyethylene-co-propyleneglycol. Hydrophobic polymer block (B) is an insoluble, superiorly biocompatible, and bio-degradable polymer, and its examples include polyester, polyanhydride, polyamino acid, polyorthoester or polyphosphazene, etc., preferably polylactide, polyglycolide, polycaprolactone, polydioxane-2-one, polylactic-co-glycolide, polylactic-co-dioxane-2-one, polylactic-co-caprolactone, polyglycolic-co-caprolactone, etc. As the above polymer, it is preferable to use a compound having the average number molecular weight of 200 to 60,000.

Surfactants may be used in 1,000 or less weight ratio to a 10-hydroxycamptothecin, preferably 1 to 500 weight ratio, and more preferably 10 to 250 weight ratio.

When the present formulation composition is prepared as a lyophilized formulation, a caking agent for lyophilization, such as mannitol, sorbitol or lactose, can be added thereto.

The present formulation composition may further contain pharmaceutically acceptable excipient, and hydrophobic oil such as tocopherol, benzylbenzoate, sesame seed oil, castor oil, soybean oil, cotton seed oil, triglyceride, etc. can be added thereto in order to delay exposure of the present composition to an aqueous solution when the composition is diluted with water for injection.

One embodiment of the method for preparing the present formulation composition as a solution or lyophilized formulation is as follows.

1 to 10 mg of a 10-hydroxycamptothecin and an amine compound are added to non-aqueous solvent by 1 to 100 equivalents to a camptothecin compound, and solubilized, and then surfactant is added thereto to prepare a solution. This solution is filtered with a filter having a pore size of 200 nm to remove insoluble substance, and then is sealed. As a specific example, 1 mg of SN-38 and 10 mg of tromethamine may be added to I ml of anhydrous ethanol to prepare a pure solution of orange color. Then, 500 mg of cremophore EL is added thereto to prepare a pure solution. This solution is diluted with acetonitrile, and then analyzed by HPLC to confirm that 100% of SN-38 exists in the form of a lactone, and the solubility of SN-38 is 98 weight% or more. Also, the prepared solution is kept in a refrigerator for 1 week, and then diluted with 0.9% of physiological saline to prepare 5 ml of solution. Thereafter, SN-38 is determined by HPLC to confirm that the concentration of SN-38 was 220 ug/ml, 95% or more of SN-38 existed in the form of the lactone, and the pH of the solution diluted with saline was 8.7.

Alternatively, a 10-hydroxycamptothecin compound and an amine compound are solubilized in non-aqueous polar solvent, and polymer surfactant is added thereto to prepare a solution. Next, a caking agent for lyophilization is added thereto to prepare a lyophilized formulation. As a specific example, 250 mg of copolymer (mPEG-PLA) of polyethyleneglycol (molecular weight: 2,000 daltons) and polylactic acid (molecular weight: 1,800 daltons) may be added to a solution in which SN-38 and tromethamine are solubilized in anhydrous ethanol as above, to prepare a pure solution, and then 4 ml of aqueous mannitol solution (100 mg/ml) as a caking agent for lyophilization is added thereto, which is filtered with a filter having a pore size of 200 nm, and lyophilized. This lyophilized formulation is reconstituted with 4 ml of water for injection, and then analyzed by HPLC to confirm that the concentration of SN-38 was 205 µg/ml, 92% or more of SN-38 existed in the form of a lactone, and the pH of the aqueous solution was 8.4.

In the present formulation composition, camptothecin compounds can maintain the active lactone form and the solubility of the compounds in non-aqueous polar solvent is increased. As a specific example, the solubility of SN-38 determined after suspending SN-38 in anhydrous ethanol without adding an amine compound was within the range of a few µg/ml. However, when determined after adding 10 equivalents of triethanolamine to anhydrous ethanol, the solubility of SN-38 was about 1 mg/ml, which is increased by about 500 or more times. Also, from HPLC analysis, SN-38 was in the form of a lactone, and no carboxy anion form was observed.

The pharmaceutical formulation according to the present invention can be used as an anti-cancer agent, and can comprise I to 40 mg of camptothecin compound in I ml of solution. Solution or lyophilized formulations having such concentration may be injected to treat cancer patients. In the case of injection, it is preferable that the concentration of the camptothecin compound in a diluted solution is about 0.001 to about 1.0 mg/ml, more preferably 0.1 to 0.5 mg/ml by diluting or reconstituting the present formulation composition with aqueous solution such as water for injection, physiological saline, or 5% dextrose solution, etc., and the pH is 3 to 12.

### Example 1: Improvement of the solubility of SN-38 in polar organic solvent in the presence of an amine compound

1 mg of SN-38 (manufacturer: Abatra Co., China) was put into each of six containers, and suspended in 1 ml of anhydrous ethanol. Diethanolamine was added to each of the containers by 0, 1, 5, 10, 20 and 100 equivalents to 1 equivalent of SN-38, and heated to 60 °C, and then kept at room temperature for 12 hours. After 12 hours, this solution was filtered with a filter having a pore size of 200 nm. The addition of 1 or more equivalents of diethanolamine provided a solution that was orange in color. The filtrate was analyzed by HPLC to determine the solubility of SN-38, under the following condition:

**[Table 1]**

| Item | Description |
|---|---|
| Column | VYDAC C18 Multi-ring (CAT, # 218MR54) |
| Column temperature | 25 °C |
| A (bluffer solution) | 3% v/v triethylamine (TEAA) aqueous solution |
| B (organic solvent) | Acetonitrile |
| A/B | 80/20, v/v |
| pH adjustment | 5,5(acetic acid) |
| Transfer of mobile phase | Isocratically |
| Flow rate | 1 ml/min |
| Wave length | Ex 318 nm, Em 515 nm |

SN-38 was dissolved in concentrations of 100 to 4,000 ng/ml in dimethyl sulfoxide (DMSO) solvent, from which the standard curve was obtained, and then SN-38 was quantified. The solubility results for SN-38 in the presence of diethanolamine are shown in Table 2.

**[Table 2]**

| Solubility of SN-38 for ethanol in the presence of diethanolamine | | | | | |
|---|---|---|---|---|---|
| Classification | SN-38 (mg) | Anhydrous ethanol(mℓ) | Equivalent for SN-38 of diethanol | Solubility (mg/ml) | Lacton content (%) |
| Control | 2 | 1 | 0 | 0.008 | 100 |
| Component 1 | 2 | 1 | 1 | 0.87 | 99 |
| Component 2 | 2 | 1 | 5 | 1.2 | 100 |
| Component 3 | 2 | 1 | 10 | 2 | 100 |
| Component 4 | 2 | 1 | 20 | 2 | 99 |
| Component 5 | 2 | 1 | 100 | 2 | 98 |

As shown in Table 2, the solubility of SN-38 in ethanol was 0.008 mg/ml, but the solubility was increased by adding diethanolamine, and the solubility was 1 mg/ml or more in the presence of 5 or more equivalents of diethanolamine.

### Example 2: Solubility of SN-38 in ethanol according to the kind of amine compound

As described in Example 1, 1 mg of SN-38 was suspended in 1 ml of anhydrous ethanol. Several kinds of amine compounds as shown in the following Table 3 were added to SN-38 by 10 equivalents, which was heated to 60 °C and then kept at room temperature for 12 hours. After 12 hours, the solution was filtered with a filter having a pore size of 200 nm, and then the solubility of SN-38 was determined by HPLC. The results are shown in Table 3.

**[Table 3]**

| Solubility of SN-38 for ethanol according to the kind of amine compound | | | | | |
|---|---|---|---|---|---|
| Classification | SN-38 (mg) | Anhydrous ethanol(mℓ) | Amine compound (10 equivalent) | Solubility (mg/ml) | Lacton content (%) |
| Component 3 | 1 | 1 | Diethanolamine | >1 | 100 |
| Component 6 | 1 | 1 | Triethanolamine | >1 | 100 |
| Component 7 | 1 | 1 | Tromethamine | >1 | 100 |
| Component 8 | 1 | 1 | Triethylamine | >1 | 100 |
| Component 9 | 1 | 1 | Diethylamine | >1 | 100 |
| Component 10 | 1 | 1 | N,N-dimethylethanolamine | >1 | 100 |

As shown in Table 3, when the amine compound was added to SN-38 by 10 equivalents, 1 mg/ml or more of SN-38 was dissolved, and 100 % of SN-38 existed in the form of a lactone, irrespective of the kind of amine added.

### Example 3: Solubility of SN-38 in polar organic solvent

As described in Example 1,1 mg of SN-38 was suspended in 1ml of several kinds of organic solvents as shown in Table 4. Tromethamine was added thereto by 10 equivalents to SN-38, heated to 60 °C, and then kept at room temperature for 12 hours. After 12 hours, the solution was filtered with a filter having a pore size of 200 nm, and then the solubility of SN-38 was determined by HPLC. The results are shown in Table 4.

**[Table 4]**

| Solubility of SN-38 for organic solvent | | | | | |
|---|---|---|---|---|---|
| Classification | SN-38 (mg) | Organic solvent(mℓ) | Tromethamine (equivalent) | Solubility (mg/ml) | Lacton content (%) |
| Component 3 | 1 | Anhydrous ethanol | 10 | >1 | 100 |
| Component 11 | 1 | Propylenglycol | 10 | >1 | 100 |
| Component 12 | 1 | Glycerine | 10 | >1 | 100 |
| Component 13 | 1 | PEG 300 | 10 | >1 | 100 |
| Component 14 | 1 | N-methylpyrrolidone | 10 | >1 | 100 |
| Component 15 | 1 | N,N-diacetamide | 10 | >1 | 100 |

As shown in Table 4, when tromethamine was added to SN-38 by 10 equivalents, 1 mg/ml or more of SN-38 was dissolved in all the kinds of organic solvents, and 100 % of SN-38 existed in the form of lactone.

### Example 4: Preparation of SN-38 formulation composition comprising surfactant

Several kinds of surfactants as shown in Table 5 were added to Component 7 of Example 2, by 200 times of weight of SN-38 to prepare a solution, and the component was shown in Table 5.

**[Table 5]**

| SN-38 composition comprising surfactant | | | | |
|---|---|---|---|---|
| Classification | SN-38 (mg) | Anhydrous ethanol (mℓ) | Amine compound (10 equivalent) | Surfactant (200 mg) |
| Component 16 | 1 | 1 | Tromethamine | Cremorphor EL |
| Component 17 | 1 | 1 | Tromethamine | Tween 80 |
| Component 18 | 1 | 1 | Tromethamine | Solutol HS 15 |
| Component 19 | 1 | 1 | Tromethamine | Vitamine E TPGS |
| Component 20 | 1 | 1 | Tromethamine | Poloxamer 188 |

### Example 5: Solubility of SN-38 formulation composition in water according to the content of surfactant

Cremophore EL was added to Component 7 of Example 2, at 10 to 500 times by weight of SN-38, and then diluted with injectable water, to make the total volume of 5 ml. This solution was filtered with a filter having a pore size of 200 nm, and the content of SN-38, the ratio of lactone to carboxy anion, and the change in hydrogen ion content were determined with time, while keeping the solution at room temperature. The components of the above formulation composition are shown in Table 6, and the result determined therefrom are shown in Table 7.

**[Table 6]**

| Composition for determining the content change of SN-38 by time in aqueous solution | | | | |
|---|---|---|---|---|
| Classification | SN-38 (mg) | Anhydrous ethanol(mℓ) | Tromethamine (equivalent) | Cremophore EL (mg) |
| Component 21 | 1 | 1 | 10 | 10 |
| Component 22 | 1 | 1 | 10 | 50 |
| Component 23 | 1 | 1 | 10 | 100 |
| Component 24 | 1 | 1 | 10 | 250 |
| Component 25 | 1 | 1 | 10 | 500 |

**[Table 7]**

| Effect of surfactant on the ratio of lactone to carboxy anion forms of SN-38 by time in aqueous solution | | | | | | |
|---|---|---|---|---|---|---|
| Classification | SN-38 (µg/mℓ) | Diluted solution (pH) | Lactone/carboxy anion ratio | | | |
| | | | 0.5 hr | 3 hr | 6 hr | 24 hr |
| Component 21 | 200 | 8.7 | 99/1 | 90/10 | 48/52 | 43/57 |
| Component 22 | 200 | 8.7 | 100/0 | 99/1 | 57/43 | 54/46 |
| Component 23 | 200 | 8.6 | 100/0 | 99/1 | 95/5 | 65/35 |
| Component 24 | 200 | 8.5 | 100/0 | 100/0 | 99/3 | 70/30 |
| Component 25 | 200 | 8.4 | 100/0 | 100/0 | 100/0 | 70/30 |

As shown in Table 7, the lactone form was converted to the carboxy anion form with time. In addition, the time to change from the lactone form to the carboxy anion form was delayed as the content of surfactant was increased.

### Example 6: Preparation of SN-38 soluble composition

According to the components shown in Table 8, SN-38 was suspended in organic solvent, and then an amine compound was added thereto to prepare a pure solution of orange color. Subsequently, surfactant was added thereto, and the solution was filtered with a filter of 200 nm, and stored in a glass vial.

**[Table 8]**

| SN-38 soluble composition | | | |
|---|---|---|---|
| SN-38 | Amine compound | Surfactant | Organic solvent |
| 1 mg | Tromethamine 10 mg | Cromophore EL 200 mg | Ethanol 1 mℓ |
| 1 mg | Tromethamine 10 mg | Cromophore EL 100 mg | Propyleneglycol 1 mℓ |
| 1 mg | Tromethamine 5 mg | Cromophore EL 100 mg | PEG 300 1 mℓ |
| 1 mg | Tromethamine 1 mg | Cromophore EL 250 mg | N,N-dimethylamide 0.25 mℓ |
| 1 mg | Diethanolamine 10 mg | Cromophore EL 500 mg | Ethanol 0.5 mℓ |
| 1 mg | Diethanolamme 15 mg | Tween 80 200 mg | Ethanol 1 mℓ |
| 1 mg | Diethanolamine 20 mg | Vitamin E TPGS 100 mg | Ethanol 1 mℓ |
| 1 mg | Triethanolamine 10 mg | Solutol HS 15 50 mg | Ethanol 1 mℓ |
| 1 mg | Diethylamine 2 mg | Poloxamer 188 10 mg | Ethanol 1 mℓ |

### Example 7: Scale-up of SN-38 soluble composition

500 mg of tromethamine was dissolved with 50 ml of anhydrous ethanol, and 50 ml of cremophore EL was added thereto to prepare a pure mixing solution. To this solution, 100 mg of SN-38 was added and heated to 60 °C to obtain a pure solution of orange color. The solution was kept at room temperature for I hour, and filtered with a filter having a pore size of 200 nm, and filled in glass vial by 2 ml of solution to prepare a formulation containing 2 mg of SN-38 in the concentration of 1 mg/ml per vial.

### Example 8: Preparation of lyophilized SN-38 soluble composition

12 mg of tromethamine was dissolved in 1 ml of anhydrous ethanol, and 1 mg of SN-38 was added thereto and heated to 60 °C to prepare a pure solution of orange color. Subsequently, the block copolymer of methoxypolyethyleneglycol and polylactic acid, and 200 mg of mPEG-PLA (mPEG molecular weight: 2,000 dalton, PLA molecular weight: 1,800 dalton) were added thereto to prepare a pure solution. Then, 5 ml of aqueous mannitol solution (50 mg/ml) was added thereto, mixed, filtered with a filter having a pore size of 200 nm, and lyophilized to prepare a lyophilized formulation.

Injectable water of 4 ml was added to the lyophilized vial to reconstitute the lyophilized formulation. Here, the pH of the aqueous solution was 8.4, the drug content was 242 µg/ml by HPLC analysis, and the lactone form of SN-38 was 97%.

### INDUSTRIAL APPLICABILITY

The formulation of the present invention can be used to increase the solubility of a 10-hydroxycamptothecin in non-aqueous polar solvent, with most of 10-hydroxycamptothecin existing in the active lactone form. Also, by using the present formulation additionally containing surfactant, the conversion into the inactive form was delayed when diluted or reconstituted with aqueous solution such as water for injection or physiological saline.

## Claims

1. An injectable formulation for increasing the solubility of a 10-hydroxycamptothecin compound in non-aqueous polar solvent, comprising the 10-hydroxycamptothecin compound of Formula (1a) or 7-ethyl-10-hydroxycamptothecin (SN-38): 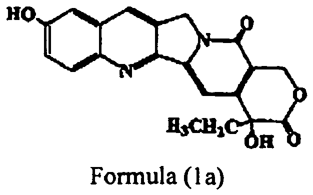 wherein the 10-hydroxycamptothecin compound of Formula (1a) is optionally substituted with alkoxy, acyloxy, hydroxy, acyl, halo, amido or cyano at 9 or 11 position of A ring or 7 position of B ring;
an amine compound whose pKₐ value is 7.4 or more, selected from the group consisting of mono-, di-, or triethanolamine, mono-, di-, or triisopropanolamine, tromethamine, triethylamine, tripropylamine, tributylamine, diethylamine, dipropylamine, dibutylamine, and mixtures thereof; and
a non-aqueous polar solvent selected from the group consisting of ethanol, propyleneglycol, liquid polyethyleneglycol having a molecular weight of 200 to 1,000 daltons glycerine, N,N-dimethylacetamide, N-methylpyrrolidone, dimethylisosorbide and mixtures thereof.

2. The formulation according to claim 1, wherein the 10-hydroxycamptothecin compound is 10-hydroxycamptothecin or 7-ethyl-10-hydroxycamptothecin (SN-38).

3. The formulation according to claim 1, comprising 1 to 100 equivalents of the amine compound to 1 equivalent of 10-hydroxycamptothecin.

4. The formulation according to claim 1, wherein the concentration of the 10-hydroxycamptothecin compound is 0.1 to 5 mg/ml.

5. The formulation according to claim 1, additionally comprising a surfactant.

6. The formulation according to claim 5, wherein the content of surfactant is 1 to 1,000 weight ratio to the 10-hydroxycamptothecin.

7. The formulation according to claim 5, wherein the surfactant is selected from the group consisting of polyoxyethylenesorbitan fatty acid ester, polyoxyethylene castor oil derivative, polyoxyethylene alkyl ether, d-α-tocopheryl polyethyleneglycol succinate, polyethyleneglycol 12-hydroxystearate, poloxamer, amphiphilic block copolymer consisting of hydrophilic polymer block (A) the hydrophobic polymer block (B), and mixture thereof, and wherein, the hydrophilic polymer block (A) is polyalkyleneglycol or derivatives thereof, polyvinylalcohol, polyvinylpyrrolidone, or polyacrylamide, and the hydrophobic polymer block (B) is polyester, polyanhydride, polyamino acid, polyorthoester, or polyphosphazene.

8. The formulation according to claim 7, comprising poloxamer or amphiphilic block copolymer consisting of hydrophilic polymer block (A) and hydrophobic polymer block (B) as a surfactant, and additionally comprising a caking agent for lyophilization, wherein the formulation is in the lyophilized form.

9. The formulation according to claim 1, additionally comprising hydrophobic oil.

10. The formulation according to claim 9, wherein hydrophobic oil is selected from the group consisting of tocopherol, benzylbenzoate, sesame seed oil, castor oil, soybean oil, cotton seed oil, triglyceride, and mixtures thereof.

11. The injectable formulation according to claim 1, wherein the formulation is diluted or reconstituted with aqueous solution so that the concentration of the 10-hydroxycamptothecin compound is in the range of 0.1 to 0.5 mg/ml, and the pH is in the range of 3 to 12.

12. The formulation according to any one of claims 1 to 11 for use as an anti-cancer agent.

## Patentansprüche

1. Injizierbare Formulierung zur Erhöhung der Löslichkeit einer 10-Hydroxycamptothecin-Verbindung in einem nicht-wässrigen, polaren Lösungsmittel, umfassend die 10-Hydroxycamptothecin-Verbindung der Formel (1a) oder 7-Ethyl-10-hydroxycamptothecin (SN-38): 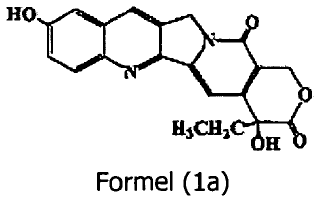 wobei die 10-Hydroxycamptothecin-Verbindung der Formel (1a) optional mit Alkoxy, Acyloxy, Hydroxy, Acyl, Halo, Amido oder Cyano an Position 9 oder 11 des A-Rings oder Position 7 des B-Rings substituiert ist;
eine Aminverbindung, deren pKₐ-Wert 7,4 oder mehr ist, ausgewählt aus der Gruppe bestehend aus Mono-, Di- oder Triethanolamin, Mono-, Di- oder Triisopropanolamin, Tromethamin, Triethylamin, Tripropylamin, Tributylamin, Diethylamin, Dipropylamin, Dibutylamin, und Mischungen davon; und
ein nicht-wässriges polares Lösungsmittel, ausgewählt aus der Gruppe bestehend aus Ethanol, Propylenglykol, flüssigem Polyethylenglykol mit einem Molekulargewicht von 200 bis 1.000 Dalton Glycerin, N,N-Dimethylacetamid, N-Methylpyrrolidon, Dimethylisosorbid, und Mischungen davon.

2. Die Formulierung gemäß Anspruch 1, wobei die 10-Hydroxycamptothecin-Verbindung 10-Hydroxycamptothecin oder 7-Ethyl-10-hydroxycamptothecin (SN-38) ist.

3. Die Formulierung gemäß Anspruch 1, umfassend 1 bis 100 Äquivalente der Aminverbindung bezogen auf 1 Äquivalent des 10-Hydroxycamptothecins.

4. Die Formulierung gemäß Anspruch 1, wobei die Konzentration der 10-Hydroxycamptothecin-Verbindung 0,1 bis 5 mg/ml ist.

5. Die Formulierung gemäß Anspruch 1, die zusätzlich ein Tensid umfasst.

6. Die Formulierung gemäß Anspruch 5, wobei der Gehalt an Tensid 1 bis 1.000 Gewichtsverhältnis bezogen auf das 10-Hydroxycamptothecin ist.

7. Die Formulierung gemäß Anspruch 5, wobei das Tensid ausgewählt ist aus der Gruppe bestehend aus Polyoxyethylen-Sorbitan-Fettsäureester, Polyoxyethylen-Rizinusöl-Derivaten, Polyoxyethylenalkylether, d-α-Tocopherylpolyethylenglykol-Succinat, Polyethylenglykol-12-hydroxystearat, Poloxamer, amphiphilischem Block-Copolymer, bestehend aus hydrophilem Polymerblock (A), hydrophobem Polymerblock (B), und Mischungen davon, und wobei der hydrophile Polymerblock (A) Polyalkylenglykol oder ein Derivat davon, Polyvinylalkohol, Polyvinylpyrrolidon oder Polyacrylamid ist, und der hydrophobe Polymerblock (B) Polyester, Polyanhydrid, Polyaminosäure, Polyorthoester oder Polyphosphazen ist.

8. Die Formulierung gemäß Anspruch 7, umfassend Poloxamer oder amphiphiles Block-Copolymer, bestehend aus hydrophilem Polymerblock (A) und hydrophobem Polymerblock (B), als Tensid, und zusätzlich umfassend einen Klumpenbildner zur Lyophilisierung, wobei die Formulierung in lyophilisierter Form ist.

9. Die Formulierung gemäß Anspruch 1, zusätzlich umfassend ein hydrophobes ÖI.

10. Die Formulierung gemäß Anspruch 9, wobei das hydrophobe ÖI ausgewählt ist aus der Gruppe bestehend aus Tocopherol, Benzylbenzoat, Sesamsamenöl, Rizinusöl, Sojabohnenöl, Baumwollsamenöl, Triglycerid, und Mischungen davon.

11. Die injizierbare Formulierung gemäß Anspruch 1, wobei die Formulierung mit wässriger Lösung verdünnt oder rekonstituiert ist, so dass die Konzentration der 10-Hydroxycamptothecin-Verbindung in einem Bereich von 0,1 bis 0,5 mg/ml ist und der pH in einem Bereich von 3 bis 12 ist.

12. Die Formulierung gemäß einem der Ansprüche 1 bis 11 zur Verwendung als Anti-Krebs-Mittel.

## Revendications

1. Formulation injectable pour augmenter la solubilité d'un composé 10-hydroxycamptothécine dans un solvant non aqueux polaire, comprenant le composé 10-hydroxycamptothécine de formule (1a) ou la 7-éthyl-10-hydroxycamptothécine (SN-38) : 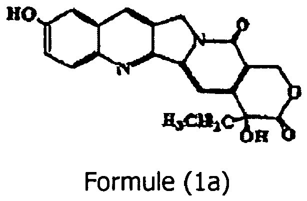 dans laquelle le composé 10-hydroxycamptothécine de formule (1a) est éventuellement substitué par un groupe alcoxy, acyloxy, hydroxy, acyle, halogéno, amido ou cyano en position 9 ou 11 du cycle A ou en position 7 du cycle B ;
un composé amine dont la valeur de pKₐ est de 7,4 ou plus, choisi dans le groupe constitué par la mono-, di-, ou triéthanolamine, la mono-, di-, ou triisopropanolamine, la trométhamine, la triéthylamine, la tripropylamine, la tributylamine, la diéthylamine, la dipropylamine, la dibutylamine, et les mélanges de celles-ci ; et
un solvant non aqueux polaire choisi dans le groupe constitué par l'éthanol, le propylène glycol, un polyéthylène glycol liquide ayant un poids moléculaire de 200 à 1 000 daltons, la glycérine, le N,N-diméthylacétamide, la N-méthylpyrrolidone, le diméthylisosorbide et les mélanges de ceux-ci.

2. Formulation selon la revendication 1, dans laquelle le composé 10-hydroxycamptothécine est la 10-hydroxycamptothécine ou la 7-éthyl-10-hydroxycamptothécine (SN-38).

3. Formulation selon la revendication 1, comprenant 1 à 100 équivalents du composé aminé pour 1 équivalent de 10-hydroxycamptothécine.

4. Formulation selon la revendication 1, dans laquelle la concentration du composé 10-hydroxycamptothécine est de 0,1 à 5 mg/ml.

5. Formulation selon la revendication 1, comprenant en outre un tensioactif.

6. Formulation selon la revendication 5, dans laquelle la teneur en tensioactif est un rapport pondéral de 1 à 1000 par rapport à la 10-hydroxycamptothécine.

7. Formulation selon la revendication 5, dans laquelle le tensioactif est choisi dans le groupe constitué par un ester d'acide gras et de sorbitan polyoxyéthyléné, un dérivé d'huile de ricin polyoxyéthyléné, un éther d'alkyle polyoxyéthyléné, un succinate de d-α-tocophéryle et de polyéthylène glycol, un 12-hydroxystéarate de polyéthylèneglycol, un poloxamère, un copolymère séquencé amphiphile constitué d'un bloc de polymère hydrophile (A), du bloc de polymère hydrophobe (B), et d'un mélange de ceux-ci, et dans lequel, le bloc de polymère hydrophile (A) est un polyalkylèneglycol ou des dérivés de celui-ci, l'alcool polyvinylique, la polyvinylpyrrolidone, ou le polyacrylamide, et le bloc de polymère hydrophobe (B) est un polyester, un polyanhydride, un acide polyaminé, un polyorthoester, ou un polyphosphazène.

8. Formulation selon la revendication 7, comprenant un poloxamère ou un copolymère séquencé amphiphile constitué d'un bloc de polymère hydrophile (A) et d'un bloc de polymère hydrophobe (B) comme tensioactif, et comprenant en outre un agent agglomérant pour la lyophilisation, dans laquelle la formulation est sous la forme lyophilisée.

9. Formulation selon la revendication 1, comprenant en outre une huile hydrophobe.

10. Formulation selon la revendication 9, dans laquelle l'huile hydrophobe est choisie dans le groupe constitué par le tocophérol, le benzoate de benzyle, l'huile de graine de sésame, l'huile de ricin, l'huile de soja, l'huile de graine de coton, un triglycéride, et les mélanges de ceux-ci.

11. Formulation injectable selon la revendication 1, dans laquelle la formulation est diluée ou reconstituée avec une solution aqueuse de sorte que la concentration du composé 10-hydroxycamptothécine est dans la gamme de 0,1 à 0,5 mg/ml, et le pH est dans la gamme de 3 à 12.

12. Formulation selon l'une quelconque des revendications 1 à 11, pour une utilisation comme agent anti-cancéreux.
